# EUROPEAN PATENT SPECIFICATION

(11) **EP 2 382 077 B1**
(45) Date of publication and mention of the grant of the patent: **16.04.2014**
(21) Application number: 10703559.4
(22) Date of filing: 27.01.2010
(51) Int. Cl.: B29C 49/48, B29C 49/52, B29C 33/42, B29C 65/78, A61M 16/04, B29L 23/00

(54) **MOLD ASSEMBLY AND METHOD FOR MANUFACTURING AN INFLATABLE BALLOON CUFF**
FORMWERKZEUG UND VERFAHREN ZUR HERSTELLUNG EINER AUFBLASBAREN BALLONMANSCHETTE
MOULE ET PROCEDE POUR LA PRODUCTION D'UNE MANCHETTE EN FORME DE BALLON GONFLABLE

(30) Priority: 29.01.2009 US 362237
(43) Date of publication of application: 02.11.2011
(73) Proprietor: Covidien LP, Mansfield, MA 02048 (US)
(72) Inventor: MACAN, Aaron, Loveland, CO 80538 (US); MEHTA, Dhairya, Skokie, IL 60077 (US); HAYMAN, Sarah, Boulder, CO 80303 (US); NEAL, Jon, Parker, CO 80138 (US); BEHLMAIER, Mark, Erie, CO 80516 (US)
(74) Representative: Kinsler, Maureen Catherine
(86) International application number: PCT/US2010/022254
(87) International publication number: WO 2010/088285

(56) References cited:
- EP-A2- 1 306 259
- WO-A1-2008/011373
- WO-A2-2008/116045
- FR-A1- 2 545 448
- JP-A- 6 226 849
- JP-A- 7 156 271
- JP-A- 55 044 832

## Description

### BACKGROUND

The present disclosure relates to medical devices, and more particularly, to airway products, such as tracheal tubes and cuffs.

This section is intended to introduce the reader to various aspects of art that may be related to various aspects of the present disclosure, which are described and/or claimed below. This discussion is believed to be helpful in providing the reader with background information to facilitate a better understanding of the various aspects of the present disclosure. Accordingly, it should be understood that these statements are to be read in this light, and not as admissions of prior art.

In the course of treating a patient, a tube or other medical device may be used to control the flow of air, food, fluids, or other substances into the patient. For example, medical devices such as tracheal tubes may be used to control the flow of one or more substances into or out of a patient. In many instances it is desirable to provide a seal between the outside of the tube or device and the interior of the passage in which the tube or device is inserted. In this way, substances can only flow through the passage via the tube or other medical device, allowing a medical practitioner to maintain control over the type and amount of substances flowing into and out of the patient.

For example, tracheal tubes may be used to control the flow of air or other gases through a patient's trachea. Such tracheal tubes may include endotracheal (ET) tubes, tracheostomy tubes, or transtracheal tubes. To seal these types of tracheal tubes, an inflatable cuff may be associated with these tubes; e.g. the endotracheal tube described in WO 2008/116045. When inflated, the cuff generally expands into the surrounding trachea to seal the tracheal passage around the tube.

Typically, cuffs are attached to tracheal tubes via an adhesive or by a heat bonding process. Because the cuffs are generally made from a relatively thin and flexible material, the cuffs may be distorted during the attachment process. For example, the cuffs may be twisted along the axis of the tube and attached to the tube in a twisted position. A twisted cuff may have an irregular inflation shape that may lead to decreased sealing efficiency or an increased incidence of wrinkling, whereby the wrinkles may create a "corkscrew" effect. In addition, a cuff may be compressed or stretched along the axis of the tube before being sealed to the tube, which may lead to a cuff that is improperly placed relative to the tube and that may have decreased sealing performance. For example, a relatively stretched cuff may have more pronounced wrinkles, which may serve as leak paths into the lungs for secretions that form at the top of the cuff. A relatively compressed cuff may have too much cuff material which will form many wrinkles when inflated and, therefore, may provide a decreased seal quality and also increases the risk that secretions will leak past the cuff into the lungs. Such secretions often contain micro-organisms that, upon aspiration by the patient, can result in complications. There remains a need in the art for an improved cuff and means of mounting the cuff to a tracheal tube that may enhance sealing performance and inhibit aspiration of secretions.

### BRIEF DESCRIPTION OF THE DRAWINGS

Advantages of the disclosure may become apparent upon reading the following detailed description and upon reference to the drawings in which:
Fig. 1 illustrates an endotracheal tube with an inflatable balloon cuff with indicators on distal and proximal collar regions in the cuff in accordance with embodiments;
Fig. 2 illustrates an exemplary balloon cuff in a twisted conformation prior to being correctly adhered to a tube according to embodiments;
Fig. 3A illustrates the balloon cuff of Fig. 2 with the indicators aligned along an X-ray line on the tube;
Fig. 3B is a cross-sectional view of the cuff and tube of Fig. 3A;
Fig. 4 illustrates an exemplary balloon cuff in a compressed conformation prior to being correctly adhered to a conduit according to embodiments;
Fig. 5 illustrates an exemplary balloon cuff with indicators being aligned against a mandrel inserted through the interior of the tube according to embodiments;
Fig. 6 illustrates an exemplary balloon cuff with indicators being aligned against corresponding protrusions on the tube;
Fig. 7 depicts an embodiment of a balloon cuff with shaped protrusions;
Fig. 8 depicts an embodiment of a balloon cuff with multiple protrusions on the proximal and distal opening regions respectively;
Fig. 9 illustrates an embodiment of a balloon cuff associated with a conduit adapted to suction secretions from the top of the cuff; and
Fig. 10 depicts an exemplary mold for forming protrusions on the balloon cuff.

### DETAILED DESCRIPTION

One or more specific embodiments of the present disclosure will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

It is desirable to provide a medical balloon, such as an endotracheal cuff or other medical balloon, that may efficiently seal the passage in which the cuff is inserted so that mechanical ventilation can be used to introduce air, oxygen, other gases, or medications into the lungs. Provided herein are balloon cuffs with features that allow an operator or automatic quality system to align a cuff against a tracheal tube to minimize stretching, compressing, twisting, or out of specification axial placement of the cuff relative to the tube. Such cuffs may include raised protrusions or other markers on the wall of the cuff that may serve as alignment indicators for aligning the cuff against the tube.

Medical cuffs as provided herein may be used in conjunction with any suitable medical device. Preferably, the medical cuffs as provided herein may be used in conjunction with an endotracheal tube, a tracheostomy tube, a circuit, an airway accessory, a connector, an adapter, a filter, a humidifier, a nebulizer, or a prosthetic. Medical cuffs as provided herein may include those as disclosed in "MEDICAL DEVICE AND TECHNIQUE FOR USING THE SAME," by Macan, et al., filed on january 29, 2009.

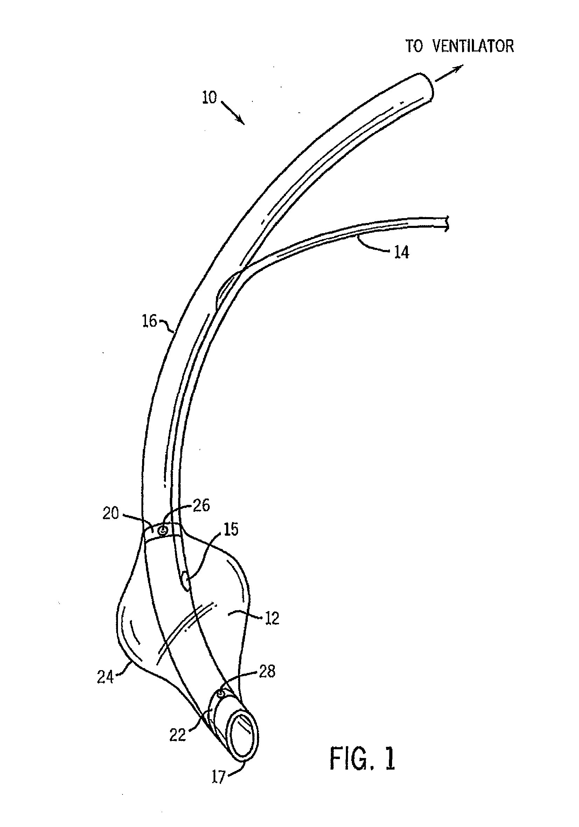

Provided herein is an exemplary cuffed endotracheal tube **10,** depicted in Fig. 1. The cuffed endotracheal tube **10** includes an inflatable medical cuff **12** that may be inflated to form a seal against the trachea wall. The medical cuff **12** is disposed on an endotracheal tube **16,** which is a conduit that is suitably sized and shaped to be inserted into a patient and allow the passage of air through the airway path of the endotracheal tube **16.** Typically, the cuff is disposed, adhesively or otherwise, towards the distal end **17** of the endotracheal tube **16.** The medical cuff **12** may, for example, be inflated and deflated via a lumen **14** in communication with the medical cuff **12,** typically through a hole or notch **15** in the endotracheal tube **16.** The medical cuff **12** includes a proximal collar region **20** and a distal collar region **22** formed in the cuff walls and sized to accommodate the endotracheal tube **16.** The proximal collar **20,** located closer to the "machine end" of the tube **16,** and a distal collar **22,** located closer to the "patient end" of the tube **16,** are typically used to mount the cuff **12** to the tube **16.** The collar regions **20** and **22** flank an inflatable region **24,** which is in fluid communication with lumen **14.** In embodiments, the cut edges of the cuff **12** may be used to position the cuff **12** relative to other features on the tube (e.g., tube tip, Murphy eye).

The medical cuff **12** may be formed from materials having suitable mechanical properties (such as puncture resistance, pin hole resistance, tensile strength), chemical properties (such as forming a suitable bond to the tube **16**), and biocompatibility. In one embodiment, the walls of the inflatable cuff **12** are made of a polyurethane having suitable mechanical and chemical properties. An example of a suitable polyurethane is Dow Pellethane^{®} 2363-90A. In another embodiment, the walls of the inflatable cuff **12** are made of a suitable polyvinyl chloride (PVC). Other suitable materials include polypropylene, polyethylene teraphthalate (PETP), low-density polyethylene (LDPE), silicone, neoprene, polyisoprene, or polyurethane (PU).

The cuff **12** includes alignment indicia or indicators **26** and **28,** which may be indicators of any type, including text, image, ink, chemical, or topographic markers. Further, although only indicia **26** and **28** are shown, it should be understood that any suitable number of alignment indicators may be present. Further, each indicator, e.g., **26** and/or **28,** may include multiple markings or members. For example, an alignment indicator may include a group of dots. As shown, indicator **26** and indicator **28** are associated with the proximal collar **26** and the distal collar **28,** respectively. In an embodiment, the alignment indicia **26** and **28** may be raised protrusions. The indicia **26** and **28** may be part of the cuff wall material and may be formed in the proximal collar **20** and distal collar **22** regions formed in the cuff wall. Alternatively, the indicia **26** and **28** may be adhesively or otherwise attached to the cuff material after assembly of the cuff **12.** The indicia **26** and **28** may be sized and shaped in any suitable configuration that does not substantially prevent the proximal collar region **20** and the distal collar region **22** from adequately sealing to the tube **16.** In embodiments, the indicia may be raised relative to the cuff walls at least enough for an operator to feel them with a fingertip, to see them with a naked eye or under magnification (*e.g*., illuminated magnification), or for a machine to sense them optically or by other modalities. In embodiments, the indicia are raised relative to the proximal collar **20** or distal collar **22** at least about 0.1 mm, at least about 0.125mm, at least about 0.5mm, at least about 1mm, at least about 2mm, or at least about 3mm. In addition, the indicia may be raised on the exterior surface of the cuff collars, i.e., the tracheal-facing surface. In addition, the indicia may be raised relative to the interior, *i.e.,* the tube adhesion surface of the cuff collars. In such embodiments, when the cuff **12** is pressed against the tube **16,** the raised portions may also be pushed against the tube **16** when the cuff **12** is adhered to the tube **16.**

In an embodiment, a colored ink may be used to form all or part of an alignment indicator or to emphasize the features of a protrusion against the cuff **12.** In other embodiments, chemical tags may be used to form the alignment indicator. For example, a UV light may be used to visualize alignment indicators that include UV or fluorescent paints.

The alignment indicators (*e.g*., indicators **26** and **28**) may be used to check cuff length and cuff twisting relative to the tube **16.** Because the positions of the proximal collar **20** and distal collar **22** regions do not change substantially with the inflation state of the cuff **12,** alignment indicators in these regions may be used to check cuff application quality in both inflated and uninflated cuffs. Specifically, because these opening regions **20** and **22** may be substantially adhered to the tube **16,** during an inflation spot check the protrusions in these regions maintain close contact with the exterior surface (*i.e*., tracheal wall-facing surface) of the tube **16.** Accordingly, indicators **26** and 28, such as protrusions, may be more easily aligned against markers on the tube **16** than any marker in the inflated region **24,** which is generally not in close proximity to the surface of the tube **16.**

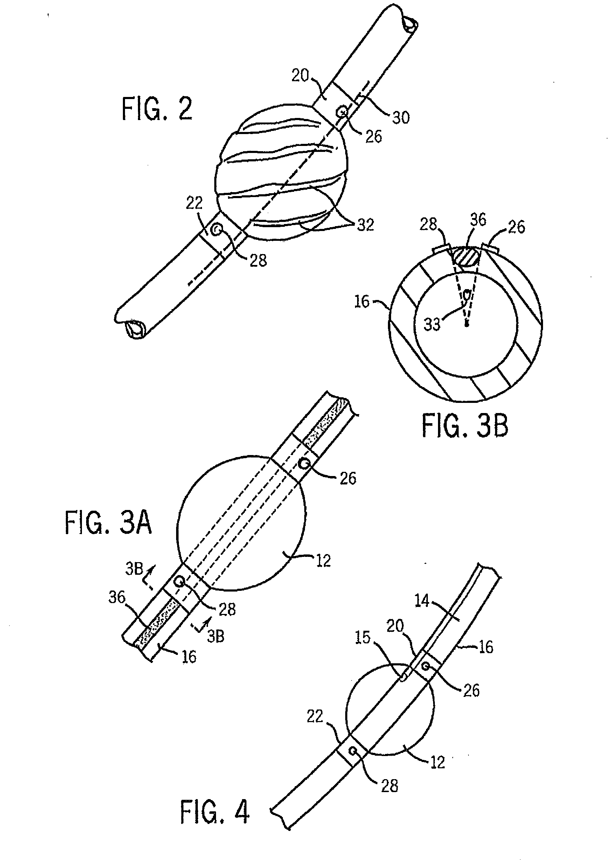

**Fig. 2** shows an exemplary cuff **12** applied to a tube **16** for an inflation spot-check prior to final adhesion of the cuff **12** to the tube **16.** As shown, the cuff **12** is twisted, and a proximal indicator **26** and distal indicator **28,** depicted as protrusions, are not aligned along an imaginary axis **30** extending through one of the indicators, for example, extending from indicator **26** as shown in **Fig. 2****,** and running along the length of the tube **16.** The twisted conformation of the cuff **12** may prevent full inflation of the cuff **12** within the trachea, which may lead to inefficient sealing. In addition, the twisted conformation may increase the formation of wrinkles **32** in the cuff **12,** which may in turn increase the availability of leak paths from the top of the cuff into the lungs. Accordingly, the rotation of the cuff **12** relative to the tube **16** may need to be altered prior to adhering the cuff **12.** The misalignment of indicators **26** and **28** may be spotted by an operator manually applying the cuff **12** to the tube **16.** Alternatively, the quality control may be performed by a machine capable of sensing a thickness in the cuff material or a visual marker at the site of each indicator (*e.g*., indicators **26** and **28**) and either alerting the operator to the misalignment or automatically aligning the indicators along the tube length based on the sensed cuff material thickness. In an embodiment, the indicators may include a marker that may be imaged by a machine to assist in locating the indicators on the cuff **12** prior to alignment. For example, the markers may include color markers, fluorescent markers, or ultraviolet light-visible markers, that, when imaged, may be manually or automatically identified to provide an indication of cuff alignment and/or placement.

In another embodiment, turning to **Fig. 3A****,** the cuff **12** may be aligned relative to the tube **16** by other features on the tube **16.** In one embodiment, the feature may be an X-ray line **36** that is marked on the tube **16,** for example by a radio opaque color, and runs along the length of tube **16.** The X-ray line may serve to provide an indication of the location of the tube **16** during X-ray visualization. In an embodiment, the X-ray line **36** may also provide the manufacturing tolerance for the rotational alignment. For example, the manufacturing tolerance may be that both indicators **26** and **28** must be touching the X-ray line **36.** In such embodiments, the effective manufacturing tolerance is the width of the X-ray line **36.** For example, in an embodiment, the manufacturing tolerance may be equal to or less than a certain angle offset of the indicators. Such an offset angle **33** may be determined by examining the alignment of indicators **26** and **28** as viewed through a cross-section of the tube **16,** as shown in **Fig. 3****B.** In **Fig. 3B****,** indicators **26** and **28** are touching the X-ray line **32** and are, thus, within a manufacturing tolerance. The offset angle **33** may be, in embodiments, equal to or less than 37°, equal to or less than 30°, or equal to or less than 27°. In addition, the offset angle **33** tolerance may be different depending on the size or shape of the cuff **12.** In other embodiments, the manufacturing tolerance may be indicated on the tube by additional alignment markers. For example, in one embodiment, a dedicated alignment line having a different placement and/or width than the X-ray line **36** may be marked on the tube **16.** In an embodiment, the line may be a different color than the X-ray line **36,** for example, the X-ray line **36** may be marked in blue while the alignment line may be pink or green.

In addition to rotational alignment, a cuff **12** may be aligned with respect to its length along the tube **16.** **Fig. 4** illustrates a balloon cuff that is in an incorrect compressed conformation prior to being adhered to a conduit. When applying a typical cuff **12** to the tube **16,** an operator may align the proximal collar **20** to a location on the tube **16** directly above notch **15** so that the inflatable region **24** is in fluid communication with the lumen **14.** However, the distal collar **22** may then be applied to the tube **16** intuitively by the operator. That is, the operator uses personal experience to guess at a correct cuff length. This may lead to variability in the cuff length based on the experience level of the operator. Accordingly, using this method, a higher number of cuffs **12** may be outside of a cuff length manufacturing tolerance, *e.g*., about 2.1 inches to about 2.3 inches or about 53mm to about 58.4mm, which may lead to waste as these cuffed tubes are discarded at later stages in the manufacturing process. In addition, quality checking procedures may include a direct measurement of cuff length with a ruler, which may be time-consuming and challenging because both the cuff **12** and the tube **16** are relatively transparent and may be difficult to differentiate from one another at the adhesion regions of the cuff **12.**

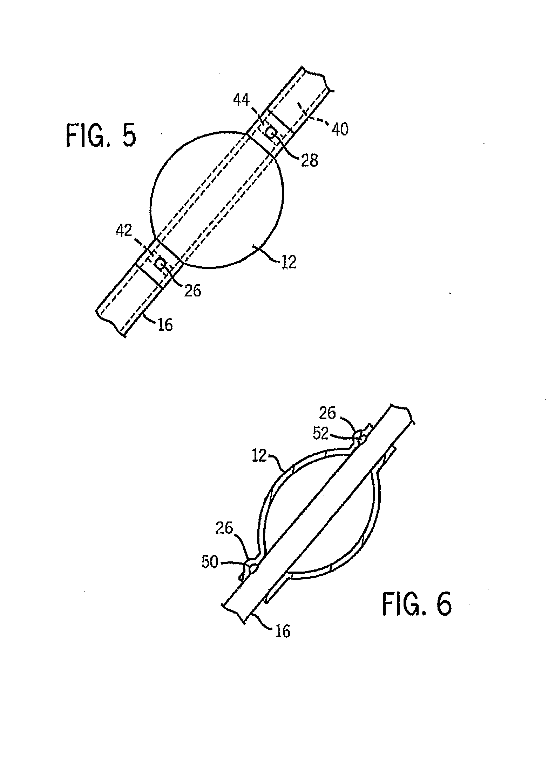

In embodiments, cuffs **12** as provided may be easily checked for relative length against the tube **16.** **Fig. 5** illustrates a cuff **12** being checked for length by aligning markers on the cuff **12** with markers on an alignment rod **40** (e.g., a mandrel) inserted through the flow path of tube **16.** The rod **40** may include alignment lines **42** and **44** that may be aligned with indicators **26** and **28** as shown. Alignment lines **42** and **44** may be brightly colored to facilitate rapid spot checks of the cuff length. The alignment lines **42** and **44** may be set to reflect the manufacturing cuff length goal, with a tolerance being built into the width of these lines. For example, the tolerance may be met if both indicators **26** and **28** are at least touching their respective lines **42** and **44.** In an embodiment, the cuff rotational alignment may be performed simultaneously with the cuff length alignment while the rod **40** is inserted in the tube **16.** In embodiments in which the rod **40** is substantially rigid, the slightly curved tube **16** may be straightened upon insertion of the rod **40,** which may allow operators to more easily perform fine rotational alignment of indicator **26** with indicator **28** along the length of tube **16.** In other embodiments, the cuff length may be verified with a ruler, a caliper, or a go/no-go gauge.

While operators may employ alignment accessories, such as rod **40,** in assisting with length alignment of the cuff **12,** the tube **16** may, in embodiments, also include indicators or features that may be used to align the cuff **12** with respect to length in addition to rotational alignment (*e.g*., the X-ray line depicted in **Fig. 3**). For example, a tube **16** may include indicators that may be aligned with markers on the cuff **12.** As shown in **Fig. 6****,** a tube **16** may include proximal protrusion **50** and distal protrusion **52** which may be aligned to proximal indicator **26** and distal indicator **28,** shown here as protrusions. Such shaped or visual alignment features on both the tube **16** and the cuff **12** may be aligned by both feel and/or sight by the operator, which may improve the ease and accuracy of the alignment.

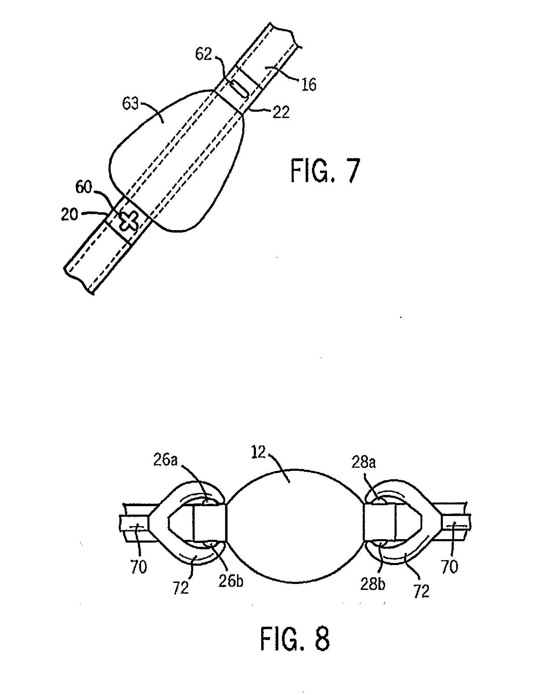

In embodiments, a cuff **12** may be bi-directional, *i.e*., the cuff **12** may be generally symmetric along an axis perpendicular to an axis through proximal collar **20** and distal collar **22** at the midpoint of the cuff length along the tube **16.** In such embodiments, the distal collar **20** may be applied to the proximal adhesion point of the tube and vice versa. However, in certain embodiments, a cuff **12** may be unidirectional and may have a specified orientation along the tube. For example, **Fig. 7** depicts an embodiment of an endotracheal tube with a tapered balloon cuff **63** in which the widest diameter is located towards the proximal end of the cuff **63.** Because cuffs **12** may be applied to the tube **16** prior to inflation, it may be difficult to determine which end of the tapered cuff **63** is the proximal collar **20** while the cuff **63** is in the uninflated state because the direction of the taper may not be evident when the tapered cuff **63** is uninflated. As provided herein, a tapered cuff **63** may include a proximal marker **60** that is different than a distal marker **62** that may facilitate the application of the proximal collar **20** to the proximal cuff adhesion point of the tube **16.** An operator may easily determine in which direction the cuff **63** should be oriented by observing shaped proximal marker **60,** which as shown, may be in a "+" shape, versus shaped distal marker **62,** which may be in a "-" shape. Any suitable combination of shaped protrusion or colored markers may be used. For example, proximal marker **60** may be green while distal marker **62** may be red and so forth. In other embodiments, a combination of shaped protrusions and colors may be used.

While alignment indicators as provided herein may be used by an operator in manually applying cuffs **12** to tubes **16,** it is envisioned that the present embodiments may also be used with automatic alignment as performed by a machine or assembly system. **Fig. 8** is an embodiment of a balloon cuff with multiple indicators, depicted as protrusions, on the proximal and distal opening regions **20** and **22,** respectively. For example, such an embodiment may be advantageous for use with an assembly machine that may provide attachment arms **70** with pincer grips **72** that may grip the cuff **12** at indicators **26a** and **26b** on the proximal end of the cuff **12** and at **28a** and **28b** at the distal end of the cuff **12.** The location of the indicators **26a, 26b** and **28a, 28b** may facilitate proper gripping of the cuff **12** by the assembly machine prior to alignment, e.g., the indicators **26a, 26b** and **28a, 28b** may provide attachment or gripping positions at which the pincer grips **72** are able to hold the cuff **12.** In such an embodiment, the alignment may then be performed by aligning the arms relative to one another at the proper length and rotation.

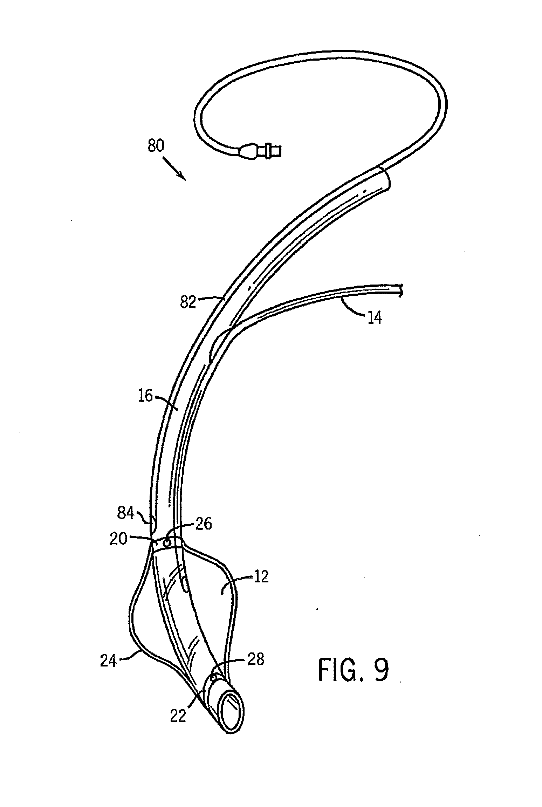

It is envisioned that the balloon cuffs **12** as provided may be used in conjunction with any suitable tracheal tube. **Fig. 9** illustrates an embodiment of a balloon cuff associated with a tracheal tube **80** that is adapted to suction secretions from the top of the cuff. The endotracheal tube **80** includes a secretion lumen **82** that has a hole **84** located above ("proximal to") the proximal shoulder of cuff **12.** The proximal collar **20** may be sealed to the tube **16** in a manner designed to move the hole **84** relative to the proximal collar **20** of the cuff **12** to facilitate aspiration of secretions that build up on this shoulder region, which may act like a shelf. While certain cuffs **12** may be aligned in an inflated state, and then adhered the tube **16,** cuffs **12** for use with such a secretion lumen may also be aligned in an uninflated state, as described herein, prior to applying the specialized seal of the proximal collar region **20** to the tube **16.** For example, the proximal collar region **20** may be shortened, either by initial design or by folding the collar region itself or the cuff wall material to decrease the distance between the cuff 12 and hole **84.**

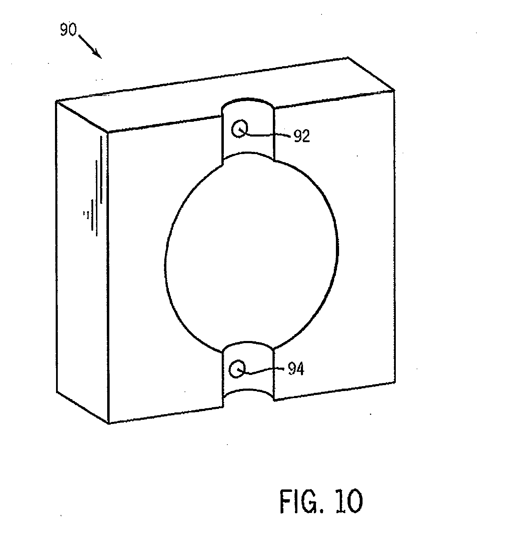

The cuffs **12** as provided are manufactured by a molding process, such as by blow molding. According to the invention, a tube, such as an extruded polyurethane tube, is loaded into a blowing machine or mold assembly, such as a cross-section of a mold assembly **90,** depicted in **Fig. 10****,** that includes dowels or shapes **92** and **94** in the mold corresponding to the desired shape of the alignment indicators **26** and **28,** e.g., protrusions, depressions. In an embodiment, shapes **92** and **94** may include injection orifices for the injection of colored markers or chemical tags to assist in visualization of the alignment indicators. In one embodiment, the mold assembly **90** is manufactured from beryllium copper and includes a horizontal split in the assembly **90** to allow opening and closing of the mold assembly **90.** In an embodiment, the mold assembly **90** may include mating symmetrical pieces that close together. The mold assembly **90** may include integrated guide pins to prevent misalignment of the two mold halves. In one embodiment, the end-portions of an extruded tube that project out from the mold are constrained to the shape and thickness of the original extruded tube by non-heat transferrable plastic holders at the ends of the mold. In one embodiment, the blow molders are model 2219H-LP blow molding machines, available from Interface Associates, that are configured to run at 1-2 bars of gas pressure. In embodiments in which the indicators (e.g., indicators **26** or **28**) may comprise shaped protrusions, the protrusions may be formed by allowing extra flash along the mold **90** parting line.

Once loaded, the mold assembly **90** is closed, and the tube is clamped at each end. The mold **90** may then be heated. The tube may be stretched and air is blown into the tube via an air conduit, such as an air hose or nozzle, connected to a source of pressurized air, such as an air pump or pre-pressurized source, to achieve a desired positive pressure within the tube and to blow out the cuff walls to the shape of the mold assembly **90.** Additional heat may be applied to the tube, such as via heating elements integral to the mold assembly to set the shape of the cuff **12.** As the heat is applied, the stretch of the tube is relaxed and the air pressure within the tube is increased. Once the desired temperature is reached it is maintained for an interval of time. Afterward, the temperature of the mold assembly is allowed to drop or is actively cooled. A vacuum is applied within the tube, which now includes the blown cuff, to release the tube and cuff from the mold assembly and the tube and cuff are removed from the mold assembly.

For example, in one embodiment, a commercially available extrusion of Dow Pellethane^{®} 2363-90AE having an inner diameter of 0.239 ± 0.005 inches (6.0706 ± 0.127 mm) and a wall thickness of 0.415 mm ± 0.007 mm may be blown to form a cuff **12** suitable for use with a 7.5 mm internal diameter (ID) endotracheal tube. The extruded tube may be cooled to room temperature and, when set, inserted into the mold assembly **90** automatically or by hand. Once loaded, the mold may be fitted into a sleeve of a blow-molding machine. The sleeve may be heated, such as by a series of ten electrical cartridges surrounding the sleeve, thereby heating the mold. In this embodiment, the mold may be heated to approximately 50° C prior to stretching or blowing the extruded tube.

An air chuck locks on to one end of the extruded tube while the other end of the extruded tube if sealed by a clamp to create an airtight seal. The extruded tube is stretched by pulling on both ends of the tube and, while stretching, nitrogen or another suitable gas or gas mixture is into the extruded tube via the air chuck to pressurize the tube to between about 1 to about 3 bars. In one embodiment, the balloon will form in the portion of the tube situated within the mold when the tube expands under pressure to make contact with the internal walls of the mold.

When the cuff is fully blown against the inner walls of the mold, the mold may be heated (such as by heating the surrounding sleeve) to between about 100° C to about 150° C and this temperature may be maintained for between about 10 to about 30 seconds. After the application of heat, the mold may be cooled to approximately 45° C, such as by pumping refrigerated water at approximately 13° C around the mold, to set the cuff. A vacuum is applied to the molded extrusion and cuff, and the extrusion and cuff are removed from the mold assembly.

The indicators, e.g., protrusions, on the wall of the cuff may serve as guides for cutting each cuff **12** during the manufacturing process. For example, after the cuff **12** is formed in a blow molding apparatus, the cuff **12** may be ready to be cut out from a longer extruded tube by a laser cutting machine. The laser may be set so that when a guide is focused on the protrusions, the cut is made at an appropriate distance from a proximal indicator **26** and a distal indicator **28.** By using the indicators as guides, each cuff **12** may be a more uniform length.

The cuff **12** may be applied to the tube **16,** which may be, for example, an extruded PVC conduit, by any suitable process. In one embodiment, the tube **16** may be inserted onto a rotating mandrel, and an operator may insert a gluing needle under the surface of the proximal collar **20** and the distal collar **22** of the cuff **12** to dispense the glue evenly.

The tracheal cuffs of the present techniques may be incorporated into systems that facilitate positive pressure ventilation of a patient, such as a ventilator. Such systems may typically include connective tubing, a gas source, a monitor, and/or a controller. The controller may be a digital controller, a computer, an electromechanical programmable controller, or any other control system.

Typically, endotracheal cuffs are inflated within a patient's trachea such that the intra cuff pressure is approximately 20-25 cm H₂O. Endotracheal cuffs utilizing inflation pressures significantly greater than 25 cm H₂O may be referred to as highpressure cuffs, while cuffs that are able to effectively seal the trachea at pressures less than 30 cm H₂O may be considered low-pressure cuffs. In certain embodiments, intra cuff inflation pressures of 10-30 cm H₂O may be used with the medical cuffs of the present techniques.

While the disclosed embodiments may be susceptible to various modifications and alternative forms, specific embodiments have been shown by way of example in the drawings and have been described in detail herein. However, it should be understood that the disclosure is not intended to be limited to the particular forms disclosed. Rather, the disclosure is to cover all modifications, equivalents, and alternatives falling within the spirit and scope of the disclosed embodiments as defined by the following appended claims.

## Claims

1. A mold assembly (90) for an inflatable balloon cuff (12) comprising: a proximal collar portion for forming a proximal collar (20) of an inflatable balloon cuff a distal collar portion for forming a distal collar (22) of the inflatable balloon cuff; a first protrusion or depression (92) in the proximal collar portion and a second protrusion or depression (94) in the distal collar portion, wherein the first protrusion or depression and the second protrusion or depression are substantially in-line with one another along an imaginary axis substantially through the first protrusion or depression and the second protrusion or depression along the length of the mold assembly.

2. The mold assembly (90) of claim 1, wherein the mold assembly is part of an extrusion blow molding system.

3. The mold assembly (90) of claim 1, wherein the mold assembly comprises beryllium copper.

4. The mold assembly (90) of claim 1, wherein at least one of the proximal collar portion or the distal collar portion comprises a dowel or recess (92, 94) adapted to form the first or second protrusion or depression (92, 94).

5. The mold assembly (90) of claim 1, wherein the first protrusion or depression (92) and the second protrusion or depression (94) are differently shaped or colored from each other.

6. The mold assembly (90) of claim 1, wherein the mold assembly comprises two symmetrical halves and/or wherein the mold assembly comprises one or more injection sites configured to apply a chemical or colorimetric marker to the inflatable balloon cuff (12).

7. A method of applying an inflatable balloon cuff (12) to a conduit (16) comprising: passing a conduit through a proximal collar (20) and a distal collar (22) in a wall of the balloon cuff; and aligning a first indicator (26) on the proximal collar with a second indicator (28) on the distal collar wherein the alignment is substantially along an axis extending through the first indicator and the second indicator along the length of the conduit.

8. The method of claim 7, comprising sealing or adhering the balloon cuff (12) to the conduit (16).

9. The method of claim 7, comprising aligning the first indicator (26) and the second indicator (28) along a set length of the conduit (16), optionally wherein aligning the first indicator and the second indicator along the set length of the conduit comprises inserting a rod (40) through the conduit, wherein the rod comprises at least one alignment marker (42, 44) corresponding to the first indicator or the second indicator.

10. The method of claim 9, wherein aligning the first indicator (26) and the second indicator (26) along the set length of the conduit (16) comprises aligning the first indicator or the second indicator to a mark or feature (50, 52, 60, 62) on the conduit.

11. A method of manufacturing an inflatable balloon cuff (12) comprising: providing a proximal collar (20) and a distal collar (22) in a wall of the balloon cuff; providing a first alignment indicator (26) on the proximal collar; and providing a second alignment indicator (28) on the distal collar.

12. The method of claim 11, comprising providing a tracheal tube (16) associated with the balloon cuff (12), wherein the tracheal tube passes through the proximal collar (20) and the distal collar (22) of the balloon cuff.

13. The method of claim 11, wherein providing the first indicator (26) on the proximal collar (20) or the second indicator (26) on the distal collar (22) comprises molding a first protrusion (26) or a second protrusion (28) into the wall of the balloon cuff, optionally wherein molding the first protrusion or the second protrusion into the wall of the balloon cuff comprises allowing extra flash in a mold assembly (90).

14. The method of claim 11, comprising cutting the cuff (12) from a longer tube using the first indicator (26) and the second indicator (28) as a cutting guide, optionally wherein cutting the cuff from the longer tube comprises optically sensing the first indicator and the second indicator, or wherein cutting the cuff from the longer tube comprises viewing the first indicator and the second indicator under a fluorescent light or an ultraviolet light.

15. The method of claim 14, wherein cutting the cuff (12) from the longer tube comprises laser cutting.

## Patentansprüche

1. Formwerkzeug (90) für eine aufblasbare Ballonmanschette (12), das aufweist: einen proximalen Bundabschnitt für das Bilden eines proximalen Bundes (20) einer aufblasbaren Ballonmanschette; einen distalen Bundabschnitt für das Bilden eines distalen Bundes (22) der aufblasbaren Ballonmanschette; einen ersten Vorsprung oder Vertiefung (92) im proximalen Bundabschnitt und einen zweiten Vorsprung oder Vertiefung (94) im distalen Bundabschnitt, wobei der erste Vorsprung oder die Vertiefung und der zweite Vorsprung oder die Vertiefung im Wesentlichen in Reihe miteinander längs einer imaginären Achse im Wesentlichen durch den ersten Vorsprung oder die Vertiefung und den zweiten Vorsprung oder die Vertiefung entlang der Länge der Formwerkzeug sind.

2. Formwerkzeug (90) nach Anspruch 1, wobei das Formwerkzeug ein Teil eines Extrudierblasformsystems ist.

3. Formwerkzeug (90) nach Anspruch 1, wobei das Formwerkzeug Beryllium-Kupfer aufweist.

4. Formwerkzeug (90) nach Anspruch 1, bei dem mindestens einer von proximalem Bundabschnitt oder distalem Bundabschnitt einen Passstift oder eine Aussparung (92, 94) aufweist, ausgebildet, um den ersten oder zweiten Vorsprung oder die Vertiefung (92, 94) zu bilden.

5. Formwerkzeug (90) nach Anspruch 1, bei dem der erste Vorsprung oder die Vertiefung (92) und der zweite Vorsprung oder die Vertiefung (94) unterschiedlich geformt sind oder eine unterschiedliche Farbe zueinander aufweisen.

6. Formwerkzeug (90) nach Anspruch 1, wobei das Formwerkzeug zwei symmetrische Hälften aufweist, und/oder wobei das Formwerkzeug eine oder mehrere Einspritzstellen aufweist, die ausgebildet sind, um eine chemische oder kolorimetrische Markierung auf die aufblasbare Ballonmanschette (12) aufzubringen.

7. Verfahren zum Anbringen einer aufblasbaren Ballonmanschette (12) an einem Kanal (16), das die folgenden Schritte aufweist: Führen eines Kanals durch einen proximalen Bund (20) und einen distalen Bund (22) in einer Wand der Ballonmanschette; und Ausrichten einer ersten Anzeige (26) am proximalen Bund mit einer zweiten Anzeige (28) am distalen Bund, wobei die Ausrichtung im Wesentlichen längs einer Achse erfolgt, die sich durch die erste Anzeige und die zweite Anzeige entlang der Länge des Kanals erstreckt.

8. Verfahren nach Anspruch 7, das den Schritt des Abdichtens oder Anhaftens der Ballonmanschette (12) am Kanal (16) aufweist.

9. Verfahren nach Anspruch 7, das den Schritt des Ausrichtens der ersten Anzeige (26) und der zweiten Anzeige (28) entlang einer eingestellten Länge des Kanals (16) aufweist, bei dem wahlweise der Schritt des Ausrichtens der ersten Anzeige und der zweiten Anzeige entlang der eingestellten Länge des Kanals den Schritt des Einsetzens eines Stabes (40) durch den Kanal aufweist, wobei der Stab mindestens eine Ausrichtungsmarkierung (42, 44) entsprechend der ersten Anzeige oder der zweiten Anzeige aufweist.

10. Verfahren nach Anspruch 9, bei dem der Schritt des Ausrichtens der ersten Anzeige (26) und der zweiten Anzeige (28) entlang der eingestellten Länge des Kanals (16) das Ausrichten der ersten Anzeige oder der zweiten Anzeige mit einer Markierung oder einem charakteristischen Merkmal (50, 52, 60, 62) am Kanal aufweist.

11. Verfahren zur Herstellung einer aufblasbaren Ballonmanschette (12), das die folgenden Schritte aufweist: Bereitstellen eines proximalen Bundes (20) und eines distalen Bundes (22) in einer Wand der Ballonmanschette; Bereitstellen einer ersten Ausrichtungsanzeige (26) am proximalen Bund; und Bereitstellen einer zweiten Ausrichtungsanzeige (28) am distalen Bund.

12. Verfahren nach Anspruch 11, das den Schritt des Bereitstellens eines Trachealtubus (16) in Verbindung mit der Ballonmanschette (12) aufweist, wobei der Trachealtubus durch den proximalen Bund (20) und den distalen Bund (22) der Ballonmanschette verläuft.

13. Verfahren nach Anspruch 11, bei dem der Schritt des Bereitstellens der ersten Anzeige (26) am proximalen Bund (20) oder der zweiten Anzeige (28) am distalen Bund (22) das Formen eines ersten Vorsprunges (26) oder eines zweiten Vorsprunges (28) in die Wand der Ballonmanschette aufweist, bei dem wahlweise das Formen des ersten Vorsprunges oder des zweiten Vorsprunges in die Wand der Ballonmanschette den Schritt des Zulassens eines zusätzlichen Austriebes in einem Formwerkzeug (90) aufweist.

14. Verfahren nach Anspruch 11, das den Schritt des Schneidens der Manschette (12) aus einem längeren Tubus bei Benutzung der ersten Anzeige (26) und der zweiten Anzeige (28) als eine Schneidführung aufweist, bei dem wahlweise das Schneiden der Manschette aus dem längeren Tubus das optische Erfassen der ersten Anzeige und der zweiten Anzeige aufweist, oder bei dem das Schneiden der Manschette aus dem längeren Tubus das Betrachten der ersten Anzeige und der zweiten Anzeige unter einem fluoreszierenden Licht oder einem ultravioletten Licht aufweist.

15. Verfahren nach Anspruch 14, bei dem der Schritt des Schneidens der Manschette (12) aus dem längeren Tubus das Laserschneiden aufweist.

## Revendications

1. Ensemble de moule (90) pour une manchette (12) en forme de ballon gonflable, comprenant:
une partie de bague proximale destinée à former une bague proximale (20) d'une manchette en forme de ballon gonflable, une partie de bague distale destinée à former une bague distale (22) de la manchette en forme de ballon; une première saillie ou dépression (92) dans la partie de bague proximale et une seconde saillie ou dépression (94) dans la partie de bague distale, dans lequel la première saillie ou dépression et la seconde saillie ou dépression sont essentiellement alignées l'une avec l'autre le long d'un axe imaginaire traversant essentiellement la première saillie ou dépression et la seconde saillie ou dépression dans le sens de la longueur de l'ensemble de moule.

2. Ensemble de moule (90) selon la revendication 1, dans lequel l'ensemble de moule fait partie d'un système de moulage par extrusion et soufflage.

3. Ensemble de moule (90) selon la revendication 1, dans lequel l'ensemble de moule comprend du cuivre au béryllium.

4. Ensemble de moule (90) selon la revendication 1, dans lequel au moins la partie de bague proximale et/ou la partie de bague distale comprend un goujon ou un creux (92, 94) adapté pour former la première ou la seconde saillie ou dépression (92, 94).

5. Ensemble de moule (90) selon la revendication 1, dans la première saillie ou dépression (92) et la seconde saillie ou dépression (94) ont une forme ou une couleur différente l'une de l'autre.

6. Ensemble de moule (90) selon la revendication 1, dans lequel l'ensemble de moule comprend deux moitiés symétriques et/ou dans lequel l'ensemble de moule comprend au moins un site d'injection configuré pour appliquer un marqueur chimique ou colorimétrique sur la manchette (12) en forme de ballon gonflable.

7. Procédé de mise en place d'une manchette (12) en forme de ballon gonflable sur un conduit (16), comprenant: le passage d'un conduit à travers une bague proximale (20) et une bague distale (22) dans une paroi de la manchette en forme de ballon; et l'alignement d'un premier indicateur (26) sur la bague proximale avec un second indicateur (28) sur la bague distale, dans lequel l'alignement est effectué essentiellement le long d'un axe s'étendant à travers le premier indicateur et le second indicateur dans le sens de la longueur du conduit.

8. Procédé selon la revendication 7, comprenant le scellement ou le collage de la manchette (12) en forme de ballon sur le conduit (16).

9. Procédé selon la revendication 7, comprenant l'alignement du premier indicateur (26) et du second indicateur (28) le long d'une longueur définie du conduit (16), facultativement dans lequel l'alignement du premier indicateur et du second indicateur le long de la longueur définie du conduit comprend l'insertion d'une tige (40) à travers le conduit, dans lequel la tige comprend au moins un marqueur d'alignement (42, 44) correspondant au premier indicateur ou au second indicateur.

10. Procédé selon la revendication 9, dans lequel l'alignement du premier indicateur (26) et du second indicateur (28) le long de la longueur définie du conduit (16) comprend l'alignement du premier indicateur ou du second indicateur avec un repère ou une caractéristique (50, 52, 60, 62) sur le conduit.

11. Procédé de fabrication d'une manchette (12) en forme de ballon, comprenant: la fourniture d'une bague proximale (20) et d'une bague distale (22) dans une paroi de la manchette en forme de ballon; la fourniture d'un premier indicateur d'alignement (26) sur la bague proximale; et la fourniture d'un second indicateur d'alignement (28) sur la bague distale.

12. Procédé selon la revendication 11, comprenant la fourniture d'une sonde trachéale (16) associée à la manchette (12) en forme de ballon, dans lequel la sonde trachéale passe à travers la bague proximale (20) et la bague distale (22) de la manchette en forme de ballon.

13. Procédé selon la revendication 11, dans lequel la fourniture du premier indicateur (26) sur la bague proximale (20) ou du second indicateur (28) sur la bague distale (22) comprend le moulage d'une première saillie (26) ou d'un seconde saillie (28) dans la paroi de la manchette en forme de ballon, facultativement dans lequel le moulage de la première saillie ou de la seconde saillie dans la paroi de la manchette en forme de ballon comprend l'admission d'un excédent de bavure dans un ensemble de moule (90).

14. Procédé selon la revendication 11, comprenant la découpe de la manchette (12) dans un tube plus long en utilisant le premier indicateur (26) et le second indicateur (28) comme guide de découpe, facultativement dans lequel la découpe de la manchette dans le tube plus long comprend la détection optique du premier indicateur et du second indicateur, ou dans lequel la découpe de la manchette dans le tube plus long comprend la visualisation du premier indicateur et du second indicateur sous une lumière fluorescente ou une lumière ultraviolette.

15. Procédé selon la revendication 14, dans lequel la découpe de la manchette (12) dans le tube plus long comprend une découpe au laser.
